# EUROPEAN PATENT APPLICATION

(11) **EP 4 542 438 A1**
(43) Date of publication of application: **23.04.2025**
(21) Application number: 24204053.3
(22) Date of filing: 01.10.2024
(51) Int. Cl.: G06F 40/131, G06F 40/157, G06F 40/174

(54) **SYSTEMS AND METHODS FOR UTILIZING DATA OBJECT REFERENCES TO ACCESS A TRANSLATED VERSION OF A VALIDATED INSTRUMENT**

(30) Priority: 18.10.2023 US 202363591384 P; 30.10.2023 US 202363594313 P; 31.05.2024 US 202418680095
(71) Applicant: Icon Clinical Research Limited, Dublin 18 D18 X5R3 (IE)
(72) Inventor: BEELER, Jeff, Cary (US); SABOL, Matt, Springfield (US); BIGLEY, John, Lincoln (US)
(74) Representative: Weldon O'Brien Ltd.

(57) **Abstract**

A method of utilizing data object references to access a translated version of a validated instrument (202, 302), the method including: retrieving (202), for a clinical study, a validated instrument, the validated instrument being drafted in a primary language. It also includes determining (304) a secondary language associated with a user; obtaining a translation key that defines an arrangement of source text within the validated instrument. The translation key is utilized (308) to generate a first translation export object that includes source text in the secondary language. The first translation export object is inserted (310) into the arrangement within the validated instrument. The validated instrument is displayed (312) with the first translation export object to a user while the user completes the validated instrument.

## Description

### RELATED APPLICATIONS

This application claims the benefit of priority to U.S. Provisional Application No. 63,594,313, filed on October 30, 2023, and to U.S. Provisional Application No. 63,591,384, filed on October 18, 2023, the disclosures of which are incorporated herein in their entireties.

### TECHNICAL FIELD

Various embodiments of the present disclosure relate generally to a translator tool, and more specifically to systems and methods for utilizing data object references to access a translated version of a validated instrument.

### BACKGROUND

Generally, clinical research involves standardization of clinical endpoints. Clinical endpoints may refer to the outcome of a clinical trial that may be statistically analyzed to help determine the efficacy of the study performed within the clinical research. Standardization of the clinical endpoint may assist in quantifying specific measures to support changes in reported data. An exemplary clinical endpoint may be questionnaires that have been validated clinically to support a regulatory submission. Building a validated questionnaire may be complex and time consuming.

Conventional techniques generally require validating a particular questionnaire for each study the questionnaire is utilized in. Validating a particular questionnaire for a particular study may takes months to perform, leading to additional time required to perform a study. Further, conventional techniques may require separately validating a respective questionnaire each time it is translated into a different language.

The present disclosure is directed to addressing challenges such as those referenced above. The background description provided herein is for the purpose of generally presenting the context of the disclosure. Unless otherwise indicated herein, the materials described in this section are not prior art to the claims in this application and are not admitted to be prior art, or suggestions of the prior art, by inclusion in this section.

### SUMMARY OF INVENTION

We describe a system as set out in claim 1, and in dependent claims 2 to 12. We also describe a method as set out in claim 13 and dependent claims 14 and 15.

In some aspects, the techniques described herein relate to a method of utilizing data object references to access a translated version of a validated instrument, the method including: retrieving, for a clinical study, a validated instrument, the validated instrument being drafted in a primary language; determining a secondary language associated with a user; obtaining a translation key that defines an arrangement of source text within the validated instrument; utilizing the translation key to generate a first translation export object that includes source text in the secondary language; extracting the source text from the validated instrument; inserting the first translation export object into the arrangement within the validated instrument; and displaying the validated instrument with the first translation export object to a user while the user completes the validated instrument.

In some aspects, the techniques described herein relate to a method, wherein the translation key includes: a language designator, an entity type, an entity identification, and an entity property path. In some aspects, the techniques described herein relate to a method, further including: navigating a database utilizing the entity property path to determine the first translation export object.

In some aspects, the techniques described herein relate to a method, wherein determining, a secondary language associated with a user further includes: accessing an International Organization Standard (ISO) 639-1 standard language code stored within a token associated with the user.

In some aspects, the techniques described herein relate to a method, wherein the first translation export object is stored in a language-independent data format.

In some aspects, the techniques described herein relate to a method, wherein the arrangement of source text within the validated instrument, corresponds to all text in the validated instrument that is in the primary language.

In some aspects, the techniques described herein relate to a method, wherein inserting the first translation export object into corresponding defined positions within the validated instrument, further includes: displaying the first translation export object within the arrangement within the validated instrument without updating or editing the validated instrument.

In some aspects, the techniques described herein relate to a method, further including: receiving as input, one or more answers to the validated instrument from the user; and causing an administrator version of the validated instrument to include the one or more answers in the primary language.

In some aspects, the techniques described herein relate to a method of utilizing data object references to provide a translated version of a validated instrument, the method including: retrieving, for a clinical study, a validated instrument, the validated instrument being drafted in a primary language; generating a translation key to identify one or more text data objects, the one or more text data objects corresponding to text in the validated instrument in the primary language; generating, based on the translation key, a translation export object that includes the one or more text data objects; obtaining a secondary language for the validated instrument; obtaining a translated text object that includes text in the secondary language corresponding to the one or more text data objects in the translation export object; and storing the translated text object and the translation key in one or more data store.

In some aspects, the techniques described herein relate to a method, wherein the translation key defines an arrangement of the one or more text data objects within the validated instrument.

In some aspects, the techniques described herein relate to a method, where obtaining a translated text object that includes text in the secondary language corresponding to the one or more text objects in the translation export object further includes: exporting the one or more text data objects of the validated instrument; and receiving translated text in the secondary language for the one or more text data objects from a secondary system.

In some aspects, the techniques described herein relate to a method, wherein the one or more data store includes a JavaScript Object Notation (JSON) document to store the translated text.

In some aspects, the techniques described herein relate to a system for utilizing data object references to access a translated version of a validated instrument, the system including: a non-transitory computer readable medium configured to store processor-readable instructions; and a processor operatively connected to the non-transitory computer readable medium, and configured to execute the instructions to perform operations including: retrieving, for a clinical study, a validated instrument, the validated instrument being drafted in a primary language; determining a secondary language associated with a user; obtaining a translation key that defines an arrangement of source text within the validated instrument; utilizing the translation key to generate a first translation export object that includes source text in the secondary language; extracting the source text from the validated instrument; inserting the first translation export object into the arrangement within the validated instrument; and displaying the validated instrument with the first translation export object to a user while the user completes the validated instrument.

In some aspects, the techniques described herein relate to a system, wherein the translation key includes: a language designator, an entity type, an entity identification, and an entity property path. In some aspects, the techniques described herein relate to a system, further including: navigating a database utilizing the entity property path to determine the first translation export object.

In some aspects, the techniques described herein relate to a system, wherein determining, a secondary language associated with a user further includes: accessing an International Organization Standard (ISO) 639-1 standard language code stored within a token associated with the user.

In some aspects, the techniques described herein relate to a system, wherein the first translation export object is stored in a language-independent data format.

In some aspects, the techniques described herein relate to a system, wherein the arrangement of source text within the validated instrument, corresponds to all text in the validated instrument that is in the primary language.

In some aspects, the techniques described herein relate to a system, wherein inserting the first translation export object into corresponding defined positions within the validated instrument, further includes: displaying the first translation export object within the arrangement within the validated instrument without updating or editing the validated instrument.

In some aspects, the techniques described herein relate to a system, further including: receiving as input, one or more answers to the validated instrument from the user; and causing an administrator version of the validated instrument to include the one or more answers in the primary language.

It is to be understood that both the foregoing general description and the following detailed description are exemplary and explanatory only and are not restrictive of the disclosed embodiments, as claimed.

### BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.
FIG. 1A depicts an exemplary environment for creating, validating, and administering an instrument, according to one or more embodiments.
FIG. 1B depicts an exemplary environment for determining translations of an instrument, according to one or more embodiments.
FIG. 2 depicts an exemplary flow diagram illustrating the utilization of data object references to store a translated version of a validated instrument, according to one or more embodiments.
FIG. 3 depicts an exemplary flow diagram illustrating the utilization of data object references to access a translated version of a validated instrument, according to one or more embodiments.
FIG. 4A-4D depict exemplary flow diagrams of workflow utilizing a translator tool, according to one or more embodiments.
FIG. 5 depicts an exemplary flow diagram illustrating a workflow for obtaining an instances of a validated instrument, according to one or more embodiments.
FIG. 6 depicts an exemplary flow diagram illustrating a workflow for obtaining access to a validated instrument, according to one or more embodiments.
FIG. 7 depicts a simplified functional block diagram of a computer, according to one or more embodiments

### DETAILED DESCRIPTION OF EMBODIMENTS

Various embodiments of the present disclosure relate generally to a translator tool, and more specifically to systems and methods for utilizing data object references to access a translated version of a validated instrument.

According to embodiments disclosed herein, systems and methods are described for utilizing data object references to provide translated versions of validated instruments. A clinical trial may have corresponding documents such as questionaries or schedules. These may be drafted and validated in a primary language. The text and text properties within a respective document may be identified through a key (e.g., JavaScript Object Notation (JSON) properties may be identified). The text in the primary language may be exported and translated to one or more secondary languages. The system may then receive the imported text in a translation file and store this in a respective storage (e.g., in a translation web service). These text files may be accessed by users of a clinical study who have locale languages different than the primary language.

According to embodiments disclosed herein, systems and methods are described for utilizing data object references and accessing translated versions of validated instruments. For a particular clinical trial, the system may receive a validated instrument (e.g., a questionnaire or a schedule) drafted in a primary language. A user of the clinical trial, e.g., a patient, administrator, trial personnel, or the like, may request to access a questionnaire or schedule. For the user, the system may determine that the user only understands a secondary language different than the primary language the validated instrument is drafted in. The system may obtain a translation key that defines text data objects within the validated instrument that require translation. The system may then utilize the translation key to obtain (e.g., utilizing JSON pathways) a translation export object that includes source text in the secondary language. The source text in the secondary language may be inserted into a defined arrangement within the validated instrument, e.g., based on the key, and displayed to a user.

Systems and methods such as the above exhibit technical benefits relative to conventional solutions. In one aspect, a particular instrument need only be validated once, rather than each translation needing a separate validation. In another aspect, the techniques above improve the efficiency for storing and accessing validated instruments in different languages. Since only translation export objects and keys need to be stored, e.g., rather than complete copies of translated instruments, an amount of storage needed to accommodate multiple translations is decreased. Accessing a translation is more efficient, relative to conventional techniques. The insertion of a translation export object may be performed upon request or need from a user, e.g., in real or near-real time.

Reference to any particular activity is provided in this disclosure only for convenience and not intended to limit the disclosure. The disclosure may be understood with reference to the following description and the appended drawings, wherein like elements are referred to with the same reference numerals.

The terminology used below may be interpreted in its broadest reasonable manner, even though it is being used in conjunction with a detailed description of certain specific examples of the present disclosure. Indeed, certain terms may even be emphasized below; however, any terminology intended to be interpreted in any restricted manner will be overtly and specifically defined as such in this Detailed Description section. Both the foregoing general description and the following detailed description are exemplary and explanatory only and are not restrictive of the features, as claimed.

In this disclosure, the term "based on" means "based at least in part on." The singular forms "a," "an," and "the" include plural referents unless the context dictates otherwise. The term "exemplary" is used in the sense of "example" rather than "ideal." The terms "comprises," "comprising," "includes," "including," or other variations thereof, are intended to cover a non-exclusive inclusion such that a process, method, or product that comprises a list of elements does not necessarily include only those elements but may include other elements not expressly listed or inherent to such a process, method, article, or apparatus. The term "or" is used disjunctively, such that "at least one of A or B" includes, (A), (B), (A and A), (A and B), etc. Relative terms, such as, "substantially" and "generally," are used to indicate a possible variation of ±10% of a stated or understood value.

It will also be understood that, although the terms first, second, third, etc. are, in some instances, used herein to describe various elements, these elements should not be limited by these terms. These terms are only used to distinguish one element from another. For example, a first contact could be termed a second contact, and, similarly, a second contact could be termed a first contact, without departing from the scope of the various described embodiments. The first contact and the second contact are both contacts, but they are not the same contact.

As used herein, the term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Terms like "provider," "medical provider," or the like generally encompass an entity, person, or organization that may seek information, resolution of an issue, or engage in any other type of interaction with a patient, e.g., to provide medical care, medical intervention or advice, or the like. Terms like "patient," "client," "participant," "study participant" or the like generally encompass any person or entity who is obtaining information, seeking resolution of an issue, or engaging in any other type of interaction with a medical provider, e.g., to receive such care, information, or the like. Terms like "user" generally encompass any person or entity that may obtain information, resolution of an issue, purchase of a product, or engage in any other type of interaction with a provider or patient, whereby the user may be the medical provider or the patient as the case may be. For example, an individual that collects data on themselves may be both a patient and a user. A user may also refer to a patient that is being assisted by a provider. Further, a user may refer to a provider entering data on behalf of a patient.

In some instances, implementing a clinical trial to conduct clinical research may include having standardization of clinical endpoints. The clinical endpoints may include outcome measures of clinical research trials. Clinical endpoints may include instruments or artifacts. An instrument or artifact may refer to an assessment that has been developed that has been shown to provide clinical meaningful outcomes based upon a certain change from the baseline of a participant. An instrument may contain specific structure and may be configured to be displayed in a manner specific to an author's intent and/or clinical trial guidelines or regulations. An artifact may include any other material or item with textual content that may be provided as part of a clinical trial. While examples and embodiments below may reference an instrument or an artifact, it should be understood that the techniques disclosed herein may be adapted to either, as well as to any other suitable textual content.

An exemplary instrument may be a questionnaire such as an electronic clinical outcome assessment ("eCOA"). An eCOA may need to be validated clinically to support regulatory submissions. Clinical validation may be a process of verifying an instrument's procedures (e.g., the set of questions) to verify that the measurements provide a clinically meaningful outcome. A clinical validation may, for example, be performed internally by a particular organization. A clinical validation may be constructed to be standardized for a validated instrument (e.g., such as a validated eCOA). A clinical validation may promote the use of a standard set of data from time point to time point and provide reliable results with a known outcome that is based upon a change over time as opposed to a random error.

The process of creating, validating, and using an instrument (e.g., an eCOA) may be time consuming. For example, the process of performing a clinical validation may take multiple months for a single eCOA to be created and validated.

Conventional systems for clinical studies may require validating an instrument for each particular study. An issue may arise that when a new study wishes to utilize a previously created validated instrument, then the instrument may need to be revalidated. Further, if a study is translated, further validations of each translation may need to be performed. For instance, context for language may affect its understood meaning, and thus even minor changes due to translation may have an impact on the way in which a reader will understand or respond to the content. In order to ensure that such variation does not materially affect the outcomes of a trial, separate validation of each translation may be required. This process may be time-consuming and waste resources for new studies. In conventional studies, questionnaires and forms may be created in each specific locale and then served to patients. This may include one or more of the following three problems: (1) only the participant's native language can be seen by the physician and the clinical staff; (2) all results are saved in the participant's native language; and (3) when reporting the saved response, it is necessary to convert the native language to a common language.

One or more embodiments of the system described herein may provide a digital platform that has the means to configure a clinically validated instrument, create screen shots for verification from the author, and store a digital version for use without changing the underlying structure of the validated instrument. The digital platform may include digital storage of an instrument, certification of validations of an instrument, translations of a particular instrument, screen shots of a particular instrument, and means to utilize a digital store for an identical representation of a validated instruments for one or more studies.

One or more embodiments of the system may offer the advantage of only requiring a single configuration of an instrument. For example, the system may offer tools to create and store a particular eCOA within the platform. The system may offer the advantage of requiring only a single validation of a created instrument. This instrument may be accessed and utilized in multiple studies while staying validated, as will be described in greater detail below. The system may offer a single certification of an instrument. If the system utilized separate instruments for each respective language, then each instrument would need to be validated or certified. This would increase the complexity, cost, and time for conducting a clinical study. Certification as used herein generally encompasses a process by which an instrument undergoes a technical validation that it conforms to an author's original electronic or paper version according to the author's wishes. This technical validation process, in embodiments, may be separate from a clinical validation of the instrument (i.e., confirming that endpoints are statistically significant), and instead refers to the verification that the look and interpretation is as the author intended with the original validated instrument.

One or more embodiments of the system may offer tools for a user and/or an organization to prepare an eCOA. The system may further offer tools to create a study, as well as a schedule that utilizes a validated eCOA. A study may refer to a research study involving human subjects that aims to evaluate the effects of a biomedical intervention (e.g., drugs, treatment, devices, etc.) on health-related outcomes. A schedule may refer to a schedule of activities that provides the tasks and milestones, along with the corresponding timeline that are critical to the execution of a study.

One or more embodiments of the system may offer a digital platform that utilizes a method for using data reference links to maintain data validation during use. As will be discussed in greater detail below, a validated instrument may be accessed for a trial session during a study. The system may allow for the transmission of a request for a particular instance of a validated instrument during the trial session. The system may be configured to receive a data reference object that is operable as a data reference link to the validated instrument that is stored in an instrument library, such that the data reference object is operable to execute the validated instrument without generating a copy of the validated instrument. The system may further allow for a Graphical User Interface ("GUI") of the trial session to operate the data reference object, such that the user device executes the validated instrument by reference.

Clinical trials may be run in one or more countries, regions, or locations which may require multiple language and variations of languages to be provided to participants of a clinical trial. Scaling the process and procedures of linguistic translations and respective validations for translations may be difficult, error prone, time consuming, and may produce unexpected results. Strict guidelines may be required to meet both regulator and copywriting requirements to ensure consistent linguistic validations. Further, in conventional implementations, each translation of an instrument may need to be separately validated, certified, stored, and maintained, which can rapidly expand the complexity, cost, and time to implement a study with participants speaking different languages.

Traditional systems may, for example, create and store translations of clinical documents by generating full translated copies of the document. For a clinical trial in which the document is a validated instrument, each translation may need to be separately validated, stored, and maintained, which may not be scalable, and may increase the overhead complexity of a trial. In an example, a trial utilizing multiple translations of an instrument may result in a branching growth of questions and responses in different languages, which can rapidly increase the complexity for maintaining consistency across the trial, expand costs for translation back to a primary language for the trial, and/or introduce difficulty in storing trial data.

One or more embodiments of the system may improve one or more aspects of a clinical trial such as the scalability, quality, and/or speed associated with utilize multiple languages and language locales within a clinical trial. The system may utilize keyed values to serve a questionnaire written in English to a participant's native language and associated dialect. The questionnaire may only be drafted once, quality checked in English, have its text extracted and sent off for translation to a locale. At a locale level, a translation may be performed and saved. Next, the instrument (e.g., the questionnaire or form), with the translated text inserted, may be served in the locale to the participant. Upon completion of the instrument, the responses may be converted back to the original language for review and reporting. An advantage is that the study team may only see the questions and answers in the original language, e.g., may be able to view translations of the participant's answers in the original language in context with the questions, also in the original language.

One or more embodiments may include a method of utilizing data object references to access a translated version of a validated instrument. The system may for example store translation metadata for a validated reference. In an example, the system may, for a validated instrument, generate a translation key that defines position or arrangement of source text and/or translation text in the validated instrument, and may generate a first translation export object that includes the source text, e.g., in the primary or preferred language for the trial. Additional translation export objects may be generated in the language of any locale, e.g., by translating the source text included in the first translation export object. Applying any translation export object with the translation key may enable inserting text of any language into the corresponding defined positions without altering the underlying validated instrument. For example, the system may, upon providing access to a validated instruments for a user linked to a non-English language, access a database and pull a data reference object of a validated instrument, access a translation export object for the non-English language, e.g., from a store of such obj ects for different languages, and apply the accessed obj ect to the validated instrument. The user may see the displayed translation while completing the validated instrument.

One or more embodiments of the system described herein) administer the instrument (e.g., the eCOA) in a language native to the participant and allow the participant to complete the study in their native language. Meanwhile, the response are saved, and the study team may review the results in the common language. In an example, the responses may be represented non-textually, e.g., a binary value for a yes/no, a number corresponding to a multiple-choice selection, etc. Such responses may be saved as a data token or key, which, when the results are viewed by the study team, enable the responses to be associated with the originally drafted text in the primary language. The results of the questions may be saved in a common language for reporting, which may assist with regulatory submissions.

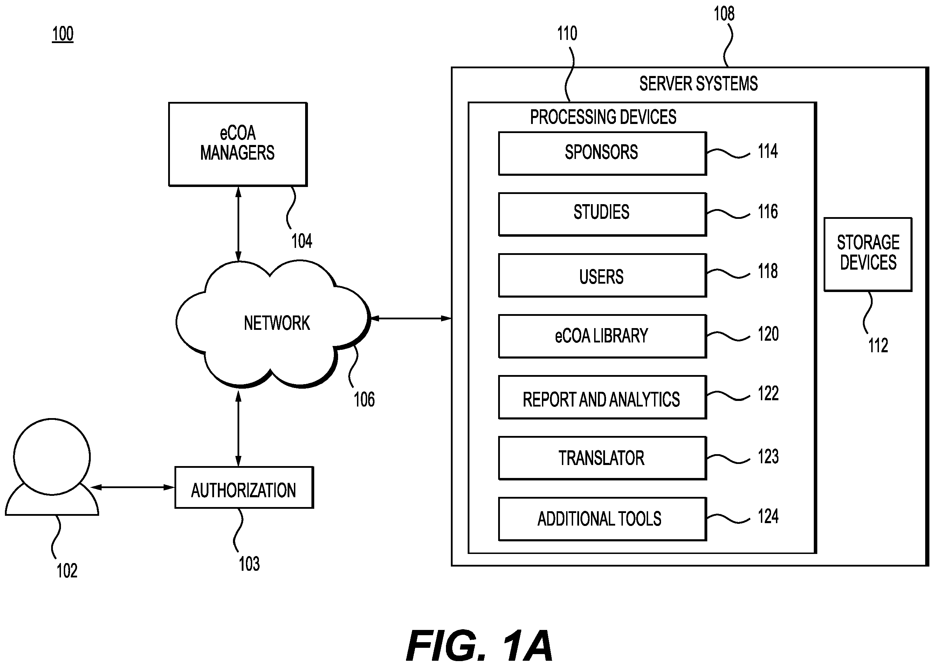

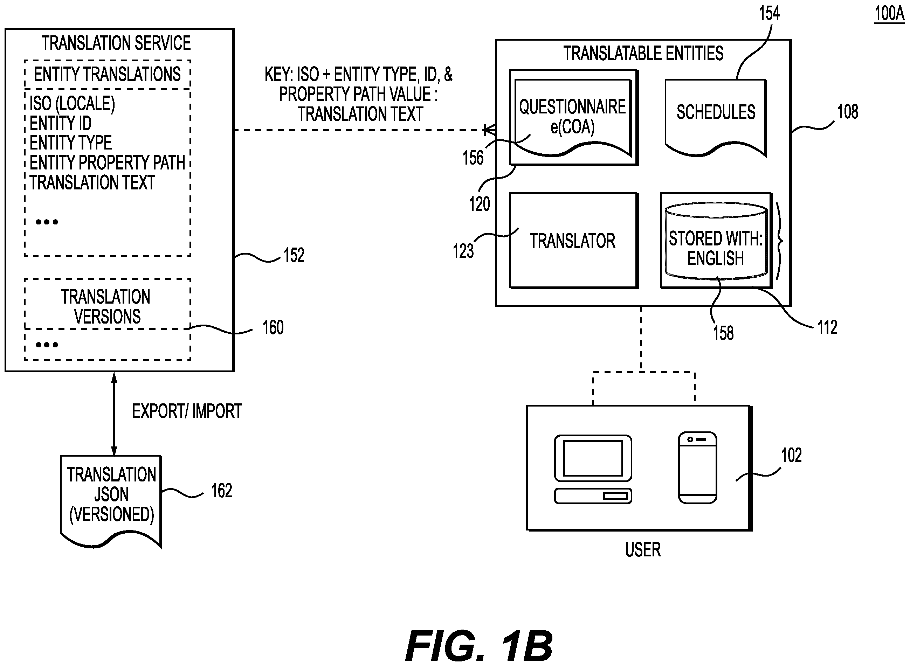

FIG. 1A depicts an exemplary environment for creating, validating, and administering an instrument, according to one or more embodiments. Environment 100 depicts an electronic network 106 that may facilitate communication with one or more eCOA Managers 104 (e.g., an administrator role that allows a user to manage eCOAs and schedules), users 102, or translation services 152 (as depicted in FIG. 1B). As used herein, an eCOA manager 104 may include or be associated with a server configured to interact with the server systems 108 described below. The eCOA manager 104 may be responsible for creating, certifying, and/or validating instruments for one or more studies. An eCOA manager 104 may further be utilized to create a study as well as a schedule for a study. The eCOA manager 104 may further include a trial server to initiate a trial session of a clinical study for one or more users 102.

Further, a user 102 may access one or more instruments through the network 106. A user 102, may for example be a patient participating within one or more studies. The user 102 may speak a language associated with the clinical study (e.g., a primary language), or a different language that the clinical study is conducted in (e.g., a secondary language). The user 102 may, for example, access an eCOA for a particular study through a GUI of a user device. The user device may be a computing device such as computer 700 described below. This may allow a user 102 to respond to and answer questions for a particular eCOA. The eCOA forms may be located in an eCOA library 120 of a server systems 108 as will be discussed in more detail below. When a user 102 accesses the system described herein, a unique token may be created that allows for the system to uniquely identify the user 102. The token may include metadata (e.g., in the standard of ISO 639-1 standard language code) of the respective user's 102 preferred language.

A user 102 may require an authorization from an authorization system 103 prior to accessing an eCOA form. For example, a user 102 may first require permission to access eCOA forms. As will be discussed in further detail below, eCOA forms may be stored in a fast healthcare interoperability resource ("FHIR") server. FHIR may refer to the set of rules and specifications required for exchanging electronic healthcare data. To access the FHIR server, the user 102 may utilize an authorization system 103, as the FHIR server may not be accessible directly from the internet. The authorization system 103 may include an identity provider ("IDP") web server that allows access to the FHIR server in which the eCOA forms are stored. The authorization system 103 may further include a firewall and a particular application programming interface ("API") (e.g., an Azure API for FHIR) for accessing the eCOA forms stored in the eCOA library 120.

A user 102, e.g., a participant, administrator, or the like, either at a clinical site or remotely may utilize a computing device (e.g., computer 700) to obtain access to a particular study. The computing device may be represented as a "client-side caller" device. This device may identify whether the participant has access to a particular study service, e.g., by performing a permission call. Permission may refer to an identifier for resources that client wants to access. The permission call may have access to a local device conducting a study. The local device may for example have access to server systems 108.

According to an exemplary embodiment of the present disclosure, the electronic network 106 may also be connected to server systems 108, which may include processing devices 110 that are configured to implement one or more modules or servers (e.g., sponsors module 114, studies module 116, users module 118, eCOA library 120, report and analytics module 122, translator 123, and additional tools 124 configured to create, certify, validate, administer, and or store one or more instruments for use within a study, according to an exemplary embodiment of the present disclosure.

The sponsors module 114 may be configured to store information related to a sponsor utilizing the server systems 108 to conduct a study. For example, the sponsors module 114 may keep track of organizations that are utilizing the server systems 108 to conduct a study. The sponsors module 114 may track each sponsor's studies, plans, and any created instruments by the particular sponsor.

The studies module 116 may include a database tracking all active and past studies performed by the server systems 108. The database within the studies module 116 may include, for example, a protocol number, a study name, a corresponding sponsor, a configuration status, and any pending actions for any studies conducted by the server systems 108. A moderator for a study may access the current results of a study through the studies module 116.

A users module 118 may be configured to store information related to patients of a study, study personnel, and system administrator. The users module 118 may further include any studies the user is participating in. The users module 118 may further include a user's full name, language(s) spoken, email address, role within the system, account status, and last login into the system described herein. Further, the users module 118 may be configured to add additional users to one or more studies. For example, an eCOA manager 104 may enter the email address, name, language, time zone, role, assigned study, and assigned site of a new user to the users module 118.

The eCOA library 120 may be configured to store one or more instruments including an eCOA. The eCOA library 120 may store the title, type of instrument, status of the instrument, amount of external version, amount of internal versions, and allow for actions to be conducted regarding an instrument. The eCOA library 120 and/or documents within the eCOA library 120 may be stored in a Fast Healthcare Interoperability Resource (FHIR) server. The eCOA library 120 may further include a "new form" function. The new form function may allow for an individual (e.g., an eCOA managers 104) to create a new eCOA form. For example, the interface may allow a user to create a display type, questions types, and sliders for a particular study. Question types may include the number, quantity, date, time, text, choice, and EuroQol (EQ)-visual analogue scale (VAS). The eCOA library 120 may further be configured to store a translation key, the translation key defining an arrangement of source text within a validated instrument.

The reports and analytics module 122 may be configured to store one or more reports and or analytics corresponding to one or more studies from the studies module 116. The reports and analytics library may include one or more data analysis tools to further analyze, graph, and organize responses to the instruments from the eCOA library 120. Further, reports may be generated that include the analysis and graphs performed. The analytics and reports may for example be accessed by the eCOA managers 104.

The translator 123 may allow for translations of the study. The translator 123 may include a questionnaire web service configured to deliver request to a system (e.g., the translation service 152 of FIG. 1B). The system may be a part of environment 100 or an external system that may be accessed through network 106. The translator 123 may be configured to export or import data objects of an instrument or artifact, while identifying a language of text requested. The translator 123 may further implement a translation key as described herein. The translator 123 may further be configured to, upon receiving translated text, insert translated text into the artifact or instrument prior to displaying the respective artifact or instrument to a user 102. FIG. 1B may depict a component diagram utilizing the translator 123. The translator 123 may for example implement JSON pathways to access particular text (e.g., the required translations) from a JSON file.

The additional tools 124 may include one or more processors or servers configured to perform function such as: developing a study, a planner for determining a schedule for a particular study, a study configuration, translations, sites, tokenization, and resources. Study configuration may refer to the phases, visits, recurring questionnaires, and their association with each. Configuration may refer to "how" instruments are assigned in a specific schedule for a participant or clinical site to complete the questionnaire. The execution of the study schedule may refer to "when" each of the events (e.g., questionnaires) will occur. The study configuration may include a calendar of the particular events.

Sites may refer to the clinical sites that are defined for each study. Each site may have one or multiple languages assigned to the configuration. The developing a study function may allow for a user to upload planned medical tests and measurements, as well as a particular timelines for all parts of the clinical study. Once a study is created, a user (e.g., an individual administering or creating the study) may assign a timeline for each phase of the study.

The server systems 108 may further include one or more storage devices 112. The storage devices 112 may be responsible for storing uploaded instruments, studies, clinical trial information, user information, reports and analytics, as well as sponsor information. Each of the modules within processing devices 110 may have access to the one or more storage devices 112.

Any of the above devices, tools and modules may be located on a device that may be connected to an electronic network 106, such as the Internet or a cloud service provider, through one or more computers, servers, and/or handheld mobile devices.

FIG. 1B depicts an exemplary environment 100A for determining translations of an instrument, according to one or more embodiments. The environment 100A of FIG. 1B may be a part of the environment 100 of FIG. 1A. The server systems 108 of FIG. 1B may be configured to interact and exchange data with a translation service 152 (e.g., though network 106 not shown). The translation services 152 may for example include an API configured to receive validated instruments and schedules from the server systems 108. Text from validated instruments, e.g., an export object generated based on a validated instrument, may for example be sent from the server systems 108 to the translation services 152 through network 106.

The environment 100A may include the server systems 108, a translation service 152, and a user device accessed by the user 102. The translation service 152 may interact with an external translator 162. The server systems 108 may, include validated instruments or artifacts (e.g., an eCOA 156 in the eCOA library 120) and created schedules 154 for clinical trials. The validated instruments (e.g., eCOAs) and schedules may, for example, be generated, stored, and saved in a first language 158 (e.g., English), within one or more storage devices 112. The validated instruments and schedules may have been previously validated. The server systems 108 may further include storage of the International Organization for Standardization (ISO) 639 codes. The ISO 639 codes may be alphabetical codes that represent the names of different languages.

The server systems 108, may utilize the translator 123 to transfer data objects (e.g., text) and/or data pertaining to documents (e.g., eCOAs and schedules) and/or corresponding metadata to the translation service 152 through network 106. The corresponding metadata may for example include an ISO 639 corresponding to a language for the translation, an entity ID, an entity type, and an entity property path. In one example, the translator 123 may, extract the text from the documents (e.g., eCOAs and schedules) prior to transferring the information to the translation services 152. The server systems 108 may only transfer the extracted text from the documents to the translation service 152, e.g., in the form of an export translation object.

The translation service 152 may receive the transfer documents or extracted document text and corresponding metadata. The translation service 152 may, upon receiving the ISO 639 code(s) from the corresponding metadata, determine what languages to perform translations of the received documents or extracted document text. For example, multiple languages may be requested for translations. Upon receiving the request, the translation service 152 may provide translations in the requested language of the received documents or document text to the server systems 108. The translation service 152 may be configured to export a translation export object, the translation export object including text for a questionnaire or schedules that requires translations, to a translator 162. The translation export object may include a property path and translated text, the property path identifying the arrangement and objects of data text objects. The translation service 152 may further be configured to import translated text data objects 160 in one or more secondary languages.

Upon performing or receiving (e.g., from a third party service) the translated text data objects 160, the translation service 152 may, for example, save the translated object(s).The translated text data objects 160 may be saved as a JavaScrip Object Notation (JSON) file. The JSON file may be transferred back to the server systems 108 through network 106 with the corresponding metadata (e.g., the ISO 639 code, entity ID, entity type, and entity property text).

The server systems 108 may for example save each of the JSON files received in a Non Structured Query Language (SQL) database. For example, the database may be a key-value databases. These may correspond to the received translated text data objects 160 from the translation services 152. The JSON files may, for example, be stored in the one or more storage devices 112 (as depicted in FIG. 1B) with corresponding keys. The keys may be configured to allow the server systems 108 to pull translated text from the JSON files and replace corresponding text in schedules and eCOAs for users.

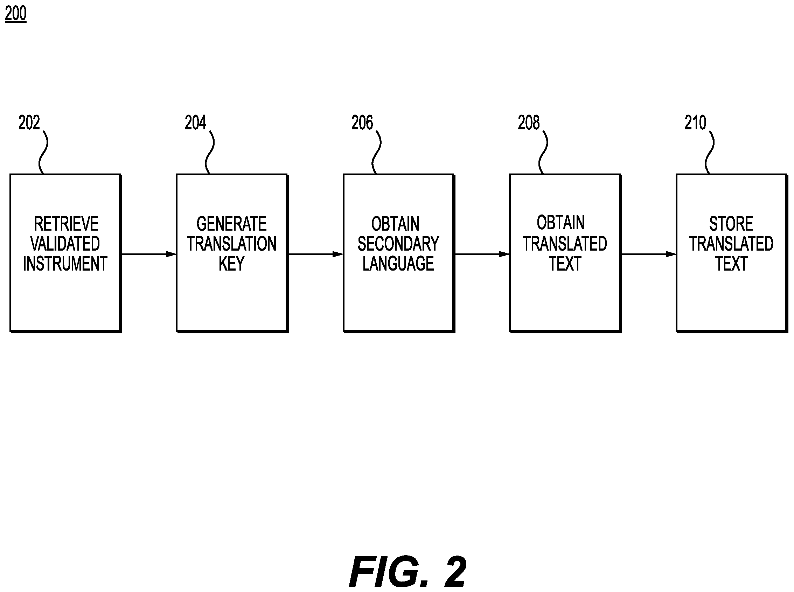

FIG. 2 depicts an exemplary flow diagram 200 illustrating the utilization of data object references to store a translated version of a validated instrument, according to one or more embodiments. The flow diagram 200 described in FIG. 2 may be performed by the environment 100 of FIG. 1A and FIG. 1B. In other examples, aspects of the flowchart described in FIG. 2 may be performed in external systems and received by the environment 100. Alternatively, the flow diagram described in flowchart 200 may be performed by any computer process system capable of administering instruments such as computer 700. Moreover, it should be understood that, in various embodiments, various steps may be added, removed, or rearranged.

Step 202 may include retrieving, for a clinical study, a validated instrument or artifact, the validated instrument being drafted in a primary language. The validated instrument or artifact may correspond to a questionnaire or schedule for a clinical study. It may be desired for the validated instruments to have translated text to administer to one or more patients of a clinical study who do not speak the primary language, but instead speak a locale language (e.g., a secondary language).

Step 204 may include generating a translation key to identify one or more text data objects, the one or more text data objects corresponding to text in the validated instrument in the primary language. The translation key may include a language designator, an entity type, an entity identification, and an entity property path. The translation key may be configured to allow the system to pull translated text from the JSON files and replace corresponding text in schedules and eCOAs for users. This may include extracting primary language text (e.g., the text data objects) and storing a way to identify the JSON property that contains the text. For example, a JSON file may include a property name and property value. The JSONPath notation may identify the JSON property that may require translations. The stored translation logic may go through the list of JSON properties that need to be translated, implement JSONPath to find the JSON property and then replace the text in the value of that property with the local language text associated with the JSON property. The translation key may define a position or arrangement of the one or more text data objects within the validated instrument. A particular text data object may correspond to a section of text within the validated instrument. For example, a title, a particular answer choice, a question, etc. may all represent individual text data objects. In some cases, multiple pages may include a single text data object (e.g., a disclaimer or a title) which are repeated throughout the validated instrument. In some embodiments, each object, even if textually identical to another, is treated separately. In other words, in some cases, text cannot be chunked so that like words or phrases are translated in aggregate. This is because, as noted above, context for even like words, phrases, or sentences may change due to translation. Separately accounting for even duplicative text may reduce a risk of needing to revalidate an instrument. In some instances, particular objects may be identified as acceptable for chunking, e.g., with a property or tag. For example, header data such as a date, clinic address, etc., which do not form the material content of an instrument but which may appear multiple times, may be acceptable to translate once in aggregate.

Step 206 may include determining a secondary language(s) for the validated instrument. The secondary language may be indicated based on ISO 639-1 standard language codes. The secondary language(s) required, which may be indicated by using ISO 639-1 standard language codes, may be stored in storage devices 112 (e.g., within the respective JSON property). For example, the secondary language may correspond to all languages associated with the users of the clinical study. In some examples, step 206 may be applied prior step 202 or 204. In some embodiments, the secondary language may be determined via a user setting or characteristic, based on property of a locale at which an instrument is being provided, via a selection by a user requesting the instrument, e.g., a trial administrator, or via any suitable process.

After step 206, the method may include generating, based on the translation key, a translation export object that includes the one or more text data objects. The particular validated instrument may be transferred from the server systems 108 to the translation services 152 of FIG. 1B. Alternatively, the one or more text data objects by themselves may be transferred from the server systems 108 to the translation services 152 of FIG. 1B with the translation key. The translation service 152 may store the way to identify the JSON property that contains the primary language text to be translated and the arrangement of the primary language text (e.g., through the translation key).

Step 208 may include obtaining a translated text object that includes text in the secondary language corresponding to the one or more text data objects in the translation export object. This may include exporting the one or more text data objects of the validated instrument to a third-party system (e.g., translator 162) and receiving translated text in the secondary language for the one or more text data objects from a secondary system. Prior to exporting the one or more text data objects of the validated instrument, the system may create a JSON file that may be used by the translator to translate the primary language text to an appropriate second language(s). The translated documents may indicate a particular language by utilizing ISO 639-1 standard language codes in the JSON property. The document may be exported and received with translations (e.g., the translated text may be imported and exported by the translation services 152). The text property may be set to the translated text in the JSON document, by the translator. Alternatively, the system may include a translator algorithm that is applied to the one or more text data objects of the validated instrument from within the translation services 152. Further, the JSON file generated for translated text, may receive text for more than one secondary language (e.g., one or more separate languages). The translation key may include JSON pathways to identify the different respective languages in the single JSON document. Further, the JSON file may be updated at later times to include additional translations of the text in additional secondary languages corresponding to the one or more text data objects in the translation export object. The JSON file may further receive additional versions of text to, for example, fix translation errors. When additional language translations are obtained at step 208, the translation key may be updated to reflect the newly received translated text. The translation key may not require regeneration when new translations are obtained.

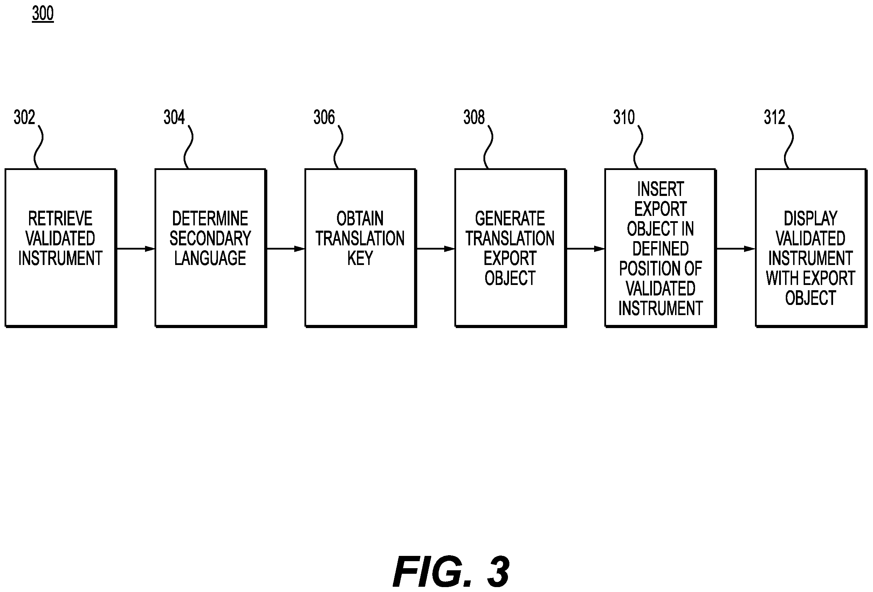

Step 210 may include storing the translated text object and the translation key in one or more data store. The translated text may be stored in a JSON document, wherein the received JSON document includes, the translated text in the secondary language and the corresponding ISO 639-1 standard language codes, may for example be saved to the translation services 152 of FIG. 1B. In some example, this translated text may be received from an external system and the file may be saved at the translation services 152. The saved translated versions of the validated instrument may be implemented by users in a clinical study as illustrated in FIG. 3

FIG. 3 depicts an exemplary flow diagram 300 illustrating the utilization of data object references to access a translated version of a validated instrument, according to one or more embodiments. The flow diagram 300 described in FIG. 3 may be performed by the environment 100 of FIG. 1A and FIG. 1B. In other examples, aspects of the flowchart described in FIG. 3 may be performed in external systems and received by the environment 100. Alternatively, the flow diagram described in flowchart 300 may be performed by any computer process system capable of administering instruments such as computer 700.

Step 302 may include retrieving, for a clinical study, a validated instrument, the validated instrument being drafted in a primary language. For example, the systems described herein (e.g., the server systems 108) may be configured to administer a schedule or eCOA (i.e., questionnaire) to a patient in a clinical trial. For example, a trial session may be initiated for a user. The trial session may include associating the user with a study. This may include assigning the user to a particular schedule and one or more validated instruments. The trial session may further associate the user with a particular language. For example, the user may have a language and corresponding ISO 639 code saved for a particular user. The user may access the trial server via an authorization system (e.g., authorization system 103), with the authorization system including (i) an identity provider web service and a system configured to access a fast healthcare interoperability resource server (e.g., where the eCOAs may be located within the eCOA library 120), and (ii) a firewall.

The system (e.g., environment 100 of FIG. 1A) may determine, via the trial server (e.g., via the processing devices 110), a validated instrument to be accessed for the trial session. For example, the validated instrument may be an eCOA. The validated instrument may for example be determined by extracting what clinical study a user is associated with. For example, the validated instrument may be extracted from the studies module 116 or a stored schedule for a clinical study. Step 302 may be initiated by a user requesting to review a particular validated instrument or artifact.

Step 304 may include determining a secondary language associated with a user. The method may include identifying the particular language required for the user based on the assigned ISO 639 code stored with a patient. For example, when a user first log's into the system described herein, as part of the user login process a token unique to the user may be created that allows the system to uniquely identify the user. This token may include metadata which includes the user's local language that may be stored. This may be stored ISO 639-1 standard language code. When retrieving the validated instrument for the user, the system may access the user token to determine the local language of the user.

Step 306 may include obtaining a translation key that defines a position of source text within the validated instrument. The translation key may include a language designator, an entity type, an entity identification, and an entity (e.g., JSON) property path. The translation key may for example have been created utilizing the techniques of step 204 of FIG. 2. The property path may assist within navigating the respective database to determine the first translation export object.

Step 308 may include utilizing the translation key to generate a first translation export object that includes source text in the secondary language. This may be implemented by the server systems 108 making a request to the translation services 152 to transfer the first translation export object that will be inserted into the instrument. Alternatively, the translation export object may already have been received by the server systems 108, and step 308 may include obtaining the translation export object from storage. This may be performed by utilizing the translation key to obtain the particular requested translation export object. In some cases, the step may include determining that a translation export object is unavailable for a particular secondary language and providing the update to a user.

The instance of the first translation export obj ect may be a reference pointer or pointer to the translated text for the validated instrument. The first translation export object may be stored in a language-independent data format. For example, it may be stored in a JSON file format. The translation key may include the JSONpath query string that may be configured to find the translated text (e.g., text stored in the JSON file from FIG. 2).

Step 310 may include inserting the first translation export object into a corresponding defined position within the validated instrument. Primary language text throughout the entire validated instrument may be replaced with the text from the first translation export object in the secondary language, e.g., as defined by the key. The position of source text within the validated instrument, corresponds to all text in the validated instrument that is in the primary language. This may include displaying the first translation export object within the corresponding defined position within the validated instrument without updating or editing the validated instrument.

Step 312 may include displaying the validated instrument with the first translation export object to a user while the user completes the validated instrument. A Graphical User Interface (GUI) of the trial session may operate the data reference object, such that a user device (e.g., device 700 as described in more detail below) executes the validated instrument by reference. In an example, a validated screen render or screenshot of the validated instrument may have had included text replaced with the translated language from the JSON file, allowing for a user to insert answers into the validated screenshot, where the validated screenshot included the translated text extracted from the non-SQL database. The user device may be a mobile cellular device. Next, the system may receive as input, one or more answers to the validated instruments from the user that may be recorded or saved. The administrator may be able to review recorded answers in the primary language, while the user answers the respective questionnaire in the secondary language.

In an example, response data entered by the user may be associated with various portions of the validated instrument, e.g., particular questions or the like. Such association may use the key generated during translation and/or a similar key object. As a result, in some embodiments, the system may be configured to associate and/or display user responses in associated with, e.g., in line with, the associated portions of the validated instrument in the primary language associated with the trial without further translation needed. Conventionally, e.g., whereby translations resulted in separate copies of instruments in different languages, viewing user responses in conjunction with the validated instrument itself may entail moving between multiple documents and/or referring to translations in order to determine which response is associated with which portion of the instrument. Instead, via one or more aspect of the translation operations discussed above, user responses may be objected in conjunction with the validated instrument directly, and without requiring further translation or reference between documents.

In an example, the system may store a library of translation objects for one or more validated instruments and/or schedules. In an example, upon identifying a requirement for a language for which a translation object for a validated instrument or schedule does not exist, the system may be configured to automatically perform method 200 to obtain a translation object for that language.

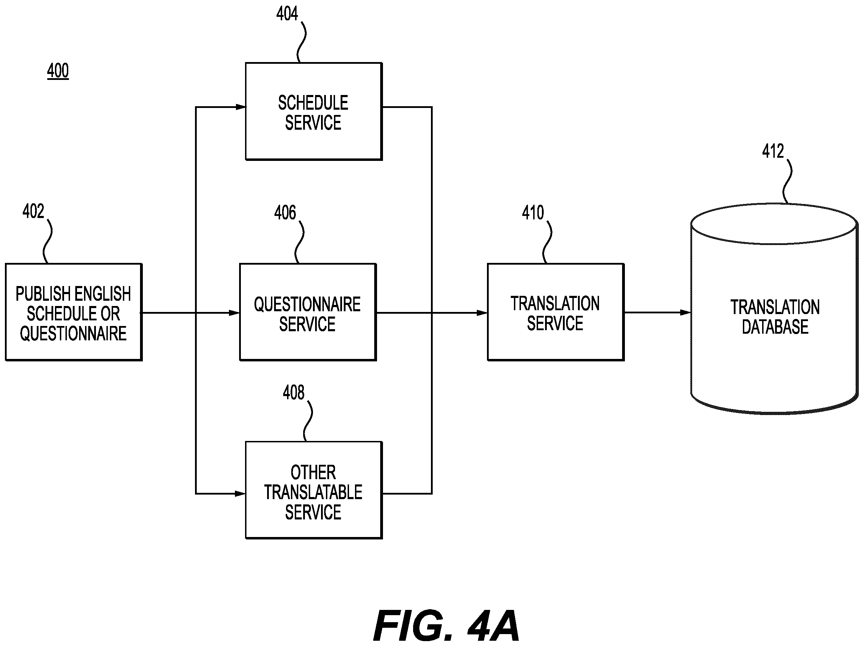

FIG. 4A-4D depict exemplary flow diagrams of workflows utilizing a translator 123, according to one or more embodiments. FIG. 4A may implement various workflows 400 of the method 200 of FIG. 2. For example, an English schedule or questionnaire 402 may be established for one or more clinical trials. Next, either the schedule service 404, questionnaire service 406, or other translatable service 408 may be sent to translation service 410 (e.g., may undergo the process of FIG. 2). The translations may then be saved to a translation database 412. These translations may then be accessed by individuals in a clinical trial (e.g., utilizing the techniques of FIG. 3).

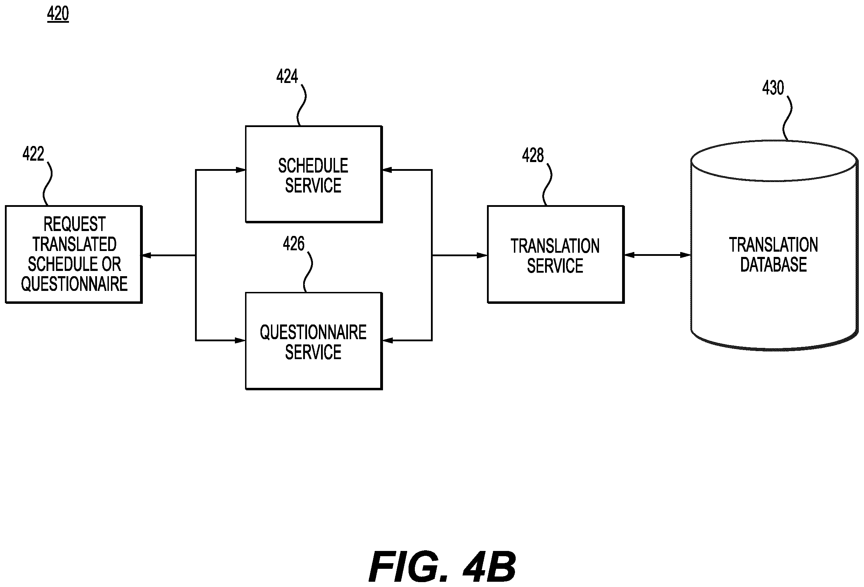

FIG. 4B may implement various workflows 420 of method 300 of FIG. 3. For example, a request for a translated schedule or questionnaire 422 may be performed by a user of a clinical study. The schedule service 424 or questionnaire service 426 may be sent for translation 428, where the translations may be accessed via a translation database 430 and delivered to the user (e.g., utilizing the techniques of FIG. 3).

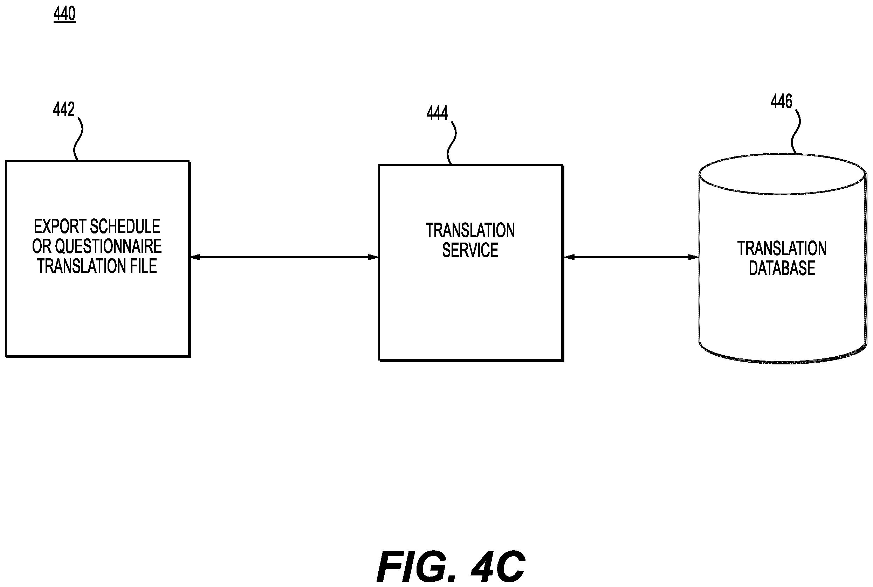

FIG. 4C may implement the process 440 of exporting a questionnaire or translation file to be stored. This may correspond to the techniques of FIG. 2 utilized to provide and store a translation of the objects for future use. For example, a schedule questionnaire translation file 442 (or text data objects from the translation file 442) may be exported to a translation service 444, wherein a translation may be created and saved to a translation database 446 for future use.

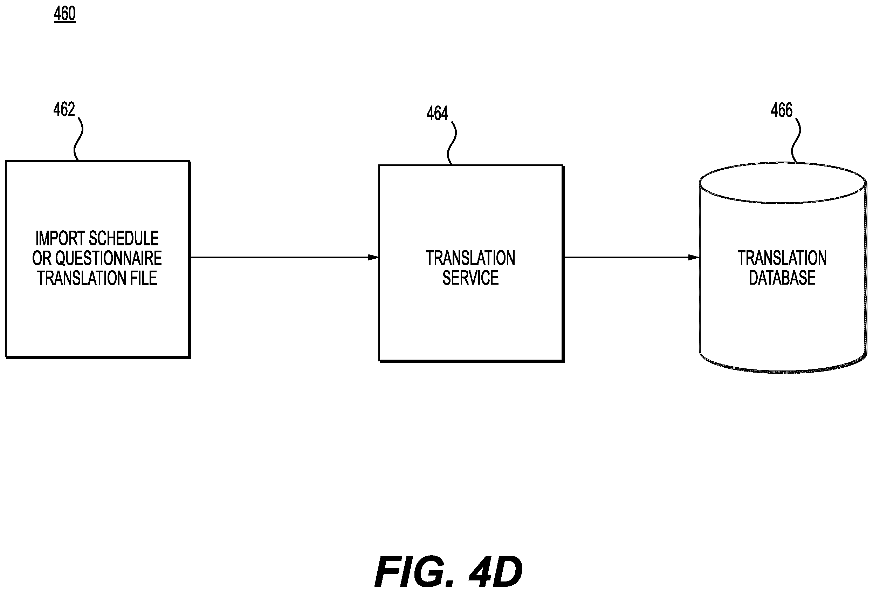

FIG. 4D may implement a process 460 of importing questionnaire or translation files. This may correspond to the techniques of FIG. 3 utilized to access a translated questionnaire or schedule. For example, a schedule questionnaire translation file 462 may be imported from a translation database 466 through a translation service 464 (e.g., the translation service 152).

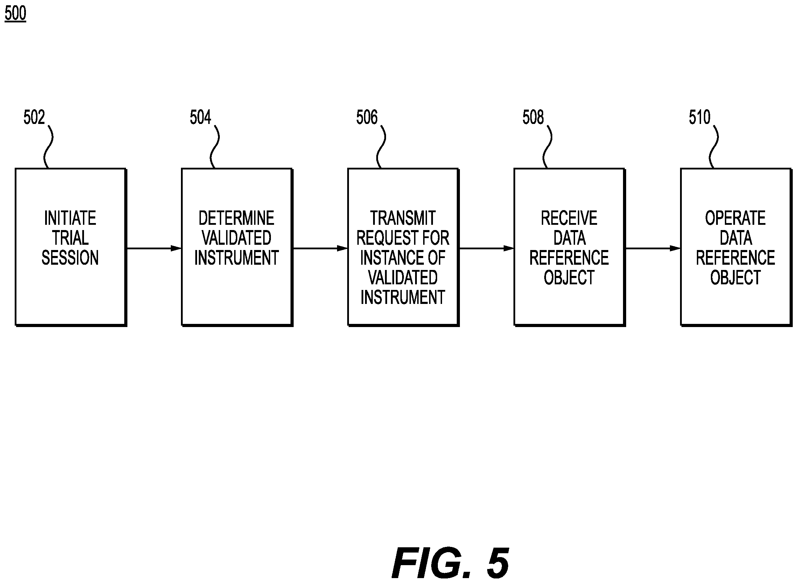

FIG. 5 depicts an exemplary flow diagram illustrating a workflow for obtaining an instance of a validated instrument, according to one or more embodiments. The flow diagram described in FIG. 5 may be performed by the environment 100 of FIG. 1A. In other examples, aspects of the flowchart described in FIG. 5 may be performed in external systems and received by the environment 100.

Alternatively, the flow diagram described in flowchart 500 may be performed by any computer process system capable of receiving image inputs such as computer 700.

At step 502, a trial session for a user may be initiated. A trial session may refer to the process and systems for a particular user to complete an instrument associated with a particular clinical study. For example, a trial session for a user may be initiated once the user has completed a portion of a clinical study. The trial session may be initiated by a provider of the clinical study. The trial session may be initiated to help standardize a clinical endpoint to meet specific measures set forth to support changes in reported data. The trial session may for example be initiated by an eCOA manager 104 accessing an electronic network 106 in order to allow a user 102 to access an eCOA from the eCOA library 120. The trial server may be initiated within the processing devices 110 of FIG. 1A. Initiating the trial session may include associating the user with a study, wherein the validated instrument is associated with the study. The user may further be associated with a particular language or locale. The user may access the trial server via an authorization system (e.g., authorization system 103), with the authorization system including (i) an identity provider web service configured to access a fast healthcare interoperability resource server (e.g., where the eCOAs may be located within the eCOA library 120), and (ii) a firewall.

At step 504, the system (e.g., environment 100 of FIG. 1A) may determine, via the trial server (e.g., via the processing devices 110), a validated instrument to be accessed for the trial session. For example, the validated instrument may be an eCOA. The validated instrument may for example be determined by extracting what clinical study a user is involved in. For example, the validated instrument may be extracted from the studies module 116 or a stored schedule for a clinical study.

At step 506, a request for an instance of the validated instrument may be transmitted to an instrument library (e.g., the eCOA library 120) and via the trial session (e.g., the processing devices 110). The instance of the validated example, may be a reference or pointer to a previously validated instrument. The instance of the validated example may be a validated screenshot of the validated instrument, allowing for the user to insert answers into the validated screenshot.

At step 508, a data reference object that is operable as a data reference link to the validated instrument stored in the instrument library (e.g., the eCOA library 120) is received from the instrument library, such that the data reference object is operable to execute the validated instrument without generating a copy of the validated instrument.

Next, upon determining that the user is associated with a secondary language (e.g., a local language), the techniques of FIG. 3 may be applied to insert translated versions of the validated instrument.

At step 510 a Graphical User Interface (GUI) of the trial session may operate the data reference object, such that a user device (e.g., device 700 as described in more detail below) executes the validated instrument by reference. This may allow one or more users (e.g., user 102) to access the validated instrument through a GUI (e.g., by accessing the server systems 108 through electronic network 106) and to interact and with and respond to the instrument. The results may then be saved to one or more storage devices (e.g., storage devices 112).

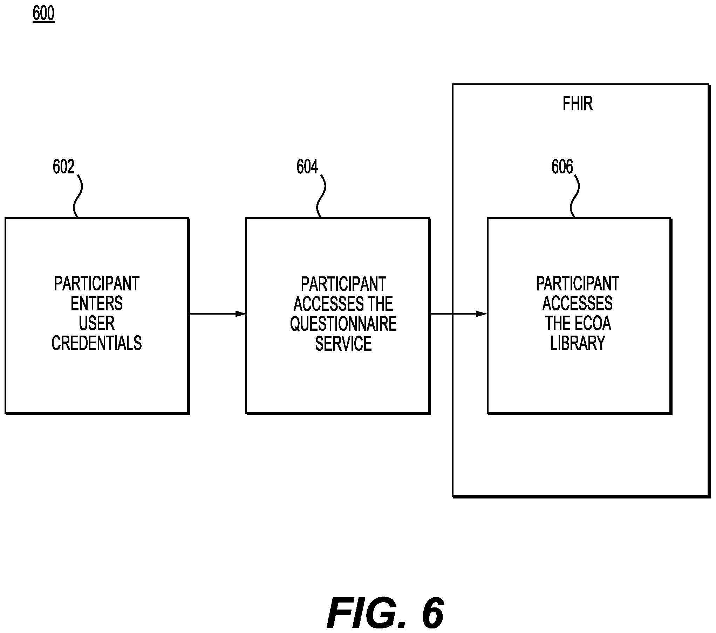

FIG. 6 depicts an exemplary flow diagram 600 illustrating a workflow for obtaining access to a validated instrument, according to one or more embodiments. The flow diagram described in FIG. 6 may be performed by the environment 100 of FIG. 1A. In other examples, aspects of the flowchart described in FIG. 6 may be performed in external systems and received by the environment 100. Alternatively, the flow diagram described in flowchart 600 may be performed by any computer process system capable of receiving image inputs such as computer 700.

At step 602, a participant may enter user credentials in order to provide initial access to a study. For example, a patient may be introduced to a clinical study. For example, a patient may be selected for a study that a particular organization operates. Upon being selected, a patient may be administered a particular care plan. The care plan may be patient specific or generated evenly for a study. The patient may, for example, go to organization site to be a participant in the clinical study. The organization site may, for example, have a medical equipment to administer the collection of samples, reading, etc. In another example, the clinical study may be administered in a remote fashion across a large geographical area with a large sample size of patients. A first step may be to provide the participant with credentials to access the study. For example, a user may be assigned (e.g., associated) with multiple credentials (e.g., a password, code, etc.) where the credential may be associated with a particular user. The credential may be assigned at an organization cite or online (e.g., through network 106). The credentials may allow a user to access one or more portions or aspects of the server systems 108 of FIG. 1A. For example, the credentials may allow for a user to access data submitted from a particular organization, data such as questionnaires from the eCOA library 120.

At step 604, the participant may access the questionnaire service (e.g., a particular organization's study). The organization study may for example be administered by an organization sponsor, where the organization sponsor administer the clinical study through a secondary organization. While accessing the organization study, the patient may follow part of a previously developed plan for the clinical trial. The plan may for example have been created and may be administered by the server systems 108.

At step 606, the participant may access the eCOA library (e.g., the eCOA library 120). For example, this access may occur upon completion of a portion of the clinical trial, whereby the research may include a clinical endpoint to meet specific measures set forth to support changes in reported data. For example, a patient (or alternatively, an administer of the clinical study on behalf of the patient) may request authorization of an instrument. For example, a user (e.g., the user 102) may utilize an authorization system (e.g., the authorization system 103) through a network to request access to the eCOA library. Upon receiving approval, a user may first complete a contract or fill out identifying information. Next, the user may obtain access to an eCOA (e.g., through the eCOA library 120). The eCOA may be updated to the user's locale language my implementing the process described in FIG. 3. The eCOA library may for example be stored in an FHIR server The eCOA may for example be a data reference object that is operable as a data reference link to the validated eCOA in the eCOA library. Through a GUI, the user may fill out the questionnaire. The answers may then be saved to storage (e.g., storage devices 112) for further analysis (e.g., by the report and analytics module 122).

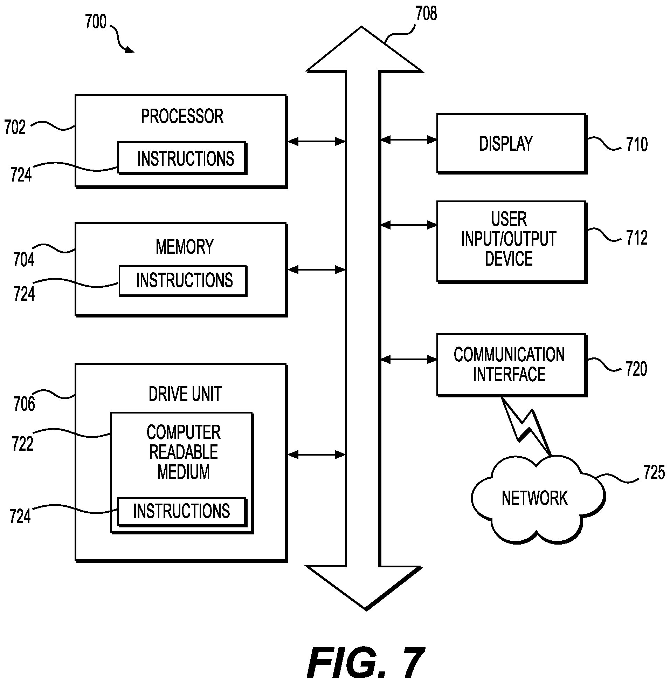

FIG. 7 depicts a simplified functional block diagram of a computer, according to one or more embodiments. The computer 700 of FIG. 7 may be utilized by a user 102 or an eCOA manager 104 to access the server systems 108 of FIG. 1A and FIG. 1B. Further the computer 700 may be utilize to execute the methods of FIG. 2, 3, 4A-4D, 5, or 6 according to exemplary embodiments of the present disclosure. One or more of a processor 702, a memory 704, a drive unit 706, an internal communication bus 708, a display 710, a under input/output ports 712, a communication interface 720, a computer readable medium 722, instructions 724, and a network 725 may communicate by any suitable means. For example, computer 700 may be configured as the server systems 108 or translation services 152, and/or another system according to exemplary embodiments of this disclosure. In various embodiments, any of the systems herein may be a computer 700 including, for example, data communication interface 720 for packet data communication. Computer 700 also may include a central processing unit (CPU) 702, in the form of one or more processors, for executing program instructions. Computer 700 may include internal communication bus 708, and storage unit 706 (such as Read-Only Memory (ROM), Hard Disk Drive (HDD), Solid-State Drive (SSD), etc.) that may store data on computer readable medium 722, although computer 700 may receive programming and data via network communications. Computer 700 may also have memory 704 (such as Random-Access Memory (RAM)) storing instructions 724 for executing techniques presented herein, although instructions 724 may be stored temporarily or permanently within other modules of computer 700 (e.g., processor 702 and/or computer readable medium 722). Computer 700 also may include input and output ports 712 and/or display 710 to connect with input and output devices such as keyboards, mice, touchscreens, monitors, displays, etc. The various system functions may be implemented in a distributed fashion on a number of similar platforms, to distribute the processing load. Alternatively, the systems may be implemented by appropriate programming of one computer hardware platform.

Program aspects of the technology may be thought of as "products" or "articles of manufacture" typically in the form of executable code and/or associated data that is carried on or embodied in a type of machine-readable medium. "Storage" type media include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer of the mobile communication network into the computer platform of a server and/or from a server to the mobile device. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

While the disclosed methods, devices, and systems are described with exemplary reference to transmitting data, it should be appreciated that the disclosed embodiments may be applicable to any environment, such as a desktop or laptop computer, an automobile entertainment system, a home entertainment system, medical equipment, etc. Also, the disclosed embodiments may be applicable to any type of Internet protocol.

It should be appreciated that in the above description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments may be used in any combination.

Thus, while certain embodiments have been described, those skilled in the art will recognize that other and further modifications may be made thereto without departing from the spirit of the invention, and it is intended to claim all such changes and modifications as falling within the scope of the invention. For example, functionality may be added or deleted from the block diagrams and operations may be interchanged among functional blocks. Steps may be added or deleted to methods described within the scope of the present invention.

The above disclosed subject matter is to be considered illustrative, and not restrictive, and the appended claims are intended to cover all such modifications, enhancements, and other implementations, which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description. While various implementations of the disclosure have been described, it will be apparent to those of ordinary skill in the art that many more implementations are possible within the scope of the disclosure. Accordingly, the disclosure is not to be restricted except in light of the attached claims and their equivalents.

## Claims

1. A system (108) for utilizing data object references to access a translated version of a validated instrument, the system comprising a non-transitory computer readable medium configured to store processor-readable instructions and a processor operatively connected to the computer readable medium and configured to execute the instructions to perform operations comprising:
retrieving (202, 302), for a clinical study, a validated instrument, the validated instrument being drafted in a primary language;
determining (206) a secondary language associated with a user;
obtaining (306) a translation key that defines an arrangement of source text within the validated instrument;
utilizing (308) the translation key to generate a first translation export object that includes source text in the secondary language;
extracting the source text from the validated instrument;
inserting (310) the first translation export object into the arrangement within the validated instrument; and
displaying (312) the validated instrument with the first translation export object to a user while the user completes the validated instrument.

2. The system of claim 1, wherein the translation key includes: a language designator, an entity type, an entity identification, and an entity property path.

3. The system of claim 2, wherein said operations further include:
navigating (204) a database utilizing the entity property path to determine the first translation export object.

4. The system of any preceding claim, wherein the operation of determining a secondary language associated with a user further includes the operation of:
accessing an International Organization Standard (ISO) 639-1 standard language code stored within a token associated with the user.

5. The system of any preceding claim, wherein the processor is configured to store the first translation export object (152) in a language-independent data format.

6. The system of any preceding claim, wherein the arrangement of source text within the validated instrument corresponds to all text in the validated instrument that is in the primary language.

7. The system of any preceding claim, wherein the processor is configured to insert the first translation export object into corresponding defined positions within the validated instrument, and to display the first translation export object within the arrangement within the validated instrument without updating or editing the validated instrument.

8. The system of any preceding claim, wherein the processor is configured to receive as an input one or more answers to the validated instrument from the user; and to cause an administrator version of the validated instrument to include the one or more answers in the primary language.

9. The system of any preceding wherein the operations include:
retrieving, for a clinical study, a validated instrument, the validated instrument being drafted in a primary language;
generating (204) a translation key to identify one or more text data objects, the one or more text data objects corresponding to text in the validated instrument in the primary language;
generating, based on said generated translation key, a translation export object that includes the one or more text data objects;
obtaining (206) a secondary language for the validated instrument; and
obtaining (208) a translated text object that includes text in the secondary language corresponding to the one or more text data objects in the translation export object; and storing (210) the translated text object and the translation key in one or more data store.

10. The system of claim 9, wherein the generated translation key defines an arrangement of the one or more text data objects within the validated instrument.

11. The system of claim 9 or claim 10, wherein said operation of obtaining a translated text object that includes text in the secondary language corresponding to the one or more text data objects in the translation export object further includes:
exporting the one or more text data objects of the validated instrument; and
receiving translated text in the secondary language for the one or more text data objects from a secondary system.

12. The system of any of claims 9 to 11, wherein the one or more data store includes a JavaScript Object Notation (JSON) document to store the translated text object.

13. A method of utilizing data object references to access a translated version of a validated instrument, the method being performed by a system comprising a non-transitory computer readable medium configured to store processor-readable instructions and a processor operatively connected to the computer readable medium and configured to execute the instructions to perform operations of the method, and the method comprising:
retrieving, for a clinical study, a validated instrument, the validated instrument being drafted in a primary language;
determining a secondary language associated with a user;
obtaining a translation key that defines an arrangement of source text within the validated instrument;
utilizing the translation key to generate a first translation export object that includes source text in the secondary language;
extracting the source text from the validated instrument;
inserting the first translation export object into the arrangement within the validated instrument; and
displaying the validated instrument with the first translation export object to a user while the user completes the validated instrument.

14. The method of claim 13, wherein the translation key includes: a language designator, an entity type, an entity identification, and an entity property path, and the method further includes navigating a database utilizing the entity property path to determine the first translation export obj ect.

15. The method of claim 14, wherein:
determining a secondary language associated with a user further includes accessing an International Organization Standard (ISO) 639-1 standard language code stored within a token associated with the user;
the first translation export object is stored in a language-independent data format;
the arrangement of source text within the validated instrument, corresponds to all text in the validated instrument that is in the primary language;
the operation of inserting the first translation export object into corresponding defined positions within the validated instrument further includes displaying the first translation export object within the arrangement within the validated instrument without updating or editing the validated instrument; and
the method further includes:
receiving as input, one or more answers to the validated instrument from the user; and
causing an administrator version of the validated instrument to include the one or more answers in the primary language.
